# EUROPÄISCHE PATENTSCHRIFT

(11) **EP 0 622 119 B1**
(45) Veröffentlichungstag und Bekanntmachung des Hinweises auf die Patenterteilung: **03.11.1999**
(21) Anmeldenummer: 94105882.8
(22) Anmeldetag: 15.04.1994
(51) Int. Cl.: B01L 9/00, G01N 35/00

(54) **System zur Bevorratung und Zurverfügungstellung von Testelementen**
System for storing and dispensing test elements
Système pour l'approvisionnement et pour la distribution d'élements de test

(30) Priorität: 23.04.1993 DE 4313252; 27.08.1993 DE 4328816
(43) Veröffentlichungstag der Anmeldung: 02.11.1994
(73) Patentinhaber: Roche Diagnostics GmbH, 68298 Mannheim (DE)
(72) Erfinder: Eikmeier, Heino Dr.,c/o Boehringer Mannheim Corp., Indianapolis, Indiana 46250 (US); Sacherer, Klaus-Dieter, D-67281 Kirchheim (DE); Schreiber, Jörg Dr., D-68542 Heddesheim (DE); Schmid, Wilfried Dr., D-68259 Mannheim (DE); Kuhr, Hans-Jürgen Dr., D-68219 Mannheim (DE)

(56) Entgegenhaltungen:
- WO-A-93/02364
- WO-A-93/07474
- WO-A-94/01780
- GB-A- 721 575
- US-A- 4 328 184
- MODERN PACKAGING, Bd.37, Nr.10, Juni 1964 Seite 131 'hinge action in polystyrene'
- PATENT ABSTRACTS OF JAPAN vol. 6, no. 129 (P-128) 15. Juli 1982 & JP-A-57 054 838 (OMRON TATEISI ELECTRONICS)

## Beschreibung

Die Erfindung betrifft ein System zur Bevorratung von Testelementen, bei dem sich zwei oder mehr Testelemente einzeln in wasserdampfdichten Kammern eines Vorratsgefäßes befinden das Vorratsgefäß aus einem starren Material gefertigt ist und die Kammern zwei Öffnungen aufweisen, die durch eine Folie wasserdampfdicht verschlossen sind.

Testelemente finden beispielsweise in der klinischen Analyse Verwendung um Inhältsstoffe in Körperflüssigkeiten qualitativ und/oder quantitativ zu detektieren. Eine besondere Bedeutung haben Testelemente, die häufig die Form von Teststreifen besitzen beim Nachweis von Glucose in Blut gewonnen. Handliche Geräte zur Blutzuckerbestimmung, in denen erfindungsgemäße Bevorratungssysteme Verwendung finden können, sind bereits weit verbreitet.

Im Stand der Technik sind Teststreifen bekannt, die einzeln in einer Folie aus Aluminium-Laminat versiegelt sind. Zur Entnahme der Testelemente wird die Folie vom Verbraucher aufgerissen und das Testelement entnommen. Ferner ist die Bevorratung von Arzneimitteln in Tablettenform bekannt. Bei den sogenannten Tabletten-Blistern befinden sich die Tabletten einzeln in Kammern, aus denen sie durch Einwirkung von mechanischem Druck freigesetzt werden können, wobei eine Versiegelung mit der Tablette durchbrochen wird.

Der geschilderte Stand der Technik besitzt für die Bevorratung und zum zur Verfügungstellen von Testelementen einige Nachteile. Eine Versiegelung von Testelementen in Aluminium-Laminaten erzeugt viel Abfall und ist für den Benutzer relativ unpraktisch, da beim Aufreißen des Aluminium-Laminates häufig Probleme auftreten. Das Entnehmen des Teststreifens aus der Verpackung erfordert eine Feinmotorik, die bei älteren und kranken Menschen häufig nicht gegeben ist. Eine automatische Entnahme von Testelementen aus biegsamen Aluminium-Laminaten ist mit einer automatischen Vorrichtung nur schwer möglich.

Die Technologie der Versiegelung von Tabletten in Blistern ist ihrerseits nicht auf Testelemente übertragbar, da Testelemente in der Regel biegsame, dünne Streifen darstellen, die nicht ohne weiteres durch die Versiegelung eines Blisters hindurchgedrückt werden können.

Im Stand der Technik ist weiterhin das Dokument WO 93/02364 bekannt, in dem ein System zur Bevorratung von Testelementen beschrieben ist. Die Testelemente befinden sich in Kammern mit einer einzigen Öffnung, die durch eine Versiegelungsfolie verschlossen ist. Zur Entnahme von Testelementen wird die Versiegelungsfolie mit einer Schneidvorrichtung durchtrennt und das in der Kammer befindliche Testelement mit einer Art Rechen, der das Testelement hintergreift, aus der Kammer herausgezogen. Weiterhin ist das Dokument WO 94/01780 bekannt, das jedoch nur gemäß Artikel 54(3) EPÜ beachtlich ist. Bei der in diesem Dokument beschriebenen Vorrichtung werden Module verwendet, die Kammern besitzen, in denen Testelemente angeordnet sind. Die Kammern weisen eine hintere Öffnung auf, durch die ein Stößel eindringt und ein in der Kammer befindliches Testelement auf einen Transporttisch schiebt. Bevor ein Herausschieben des Testelementes erfolgt, wird jedoch die die vordere Öffnung der Kammer versiegelnde Folie mit einem Schneidwerkzeug entfernt.

In dem Dokument JP 57054838 werden weiterhin Module beschrieben, die nebeneinander angeordnete Kammern aufweisen, in denen sich Testelemente befinden. Zur Entnahme der Testelemente werden diese mit einem Stößel aus den Kammern herausgeschoben. In dem Dokument wird keinerlei Versiegelung der Kammern beschrieben.

Der Erfindung lag demnach die Aufgabe zugrunde, ein System zur Bevorratung zur Verfügung zu stellen, in dem Teststreifen einzeln versiegelt vorliegen und aus dem sie auf einfache Weise, manuell oder mechanisch, einzeln entnommen werden können. Das System soll weiterhin gewährleisten, daß eine Entnahme von Teststreifen die Haltbarkeit der im System verbleibenden Teststreifen nicht wesentlich herabsetzt. Das System soll außerdem eine Deformation von Testelementen bei ihrer Entnahme weitestgehend ausschließen.

Die vorstehenden Aufgaben werden durch ein System zur Bevorratung von Testelementen gemäß Anspruch 1 gelöst. Die Erfindung betrifft außerdem ein Verfahren zum zur Verfügungstellen von Testelementen gemäß Anspruch 9.

Ein erfindungsgemäßes System beinhaltet ein Vorratsgefäß, das Kammern besitzt, die voneinander getrennt sind. Die räumliche Trennung der Kammern stellt ein wichtiges erfindungsgemäßes Merkmal dar, da aufdiese Weise der Austausch von Wasserdampf zwischen den Kammern stark reduziert ist und bei Öffnung einer Kammer zur Entnahme eines Testelements die im System verbleibenden Testelemente nicht mit wasserdampfhaltiger Umgebungsluft in Kontakt gebracht werden.

Ein weiterer, für eine einfache Entnahme eines Testelements wichtiger Aspekt des Vorratsbehältnisses ist seine Formstabilität. Die Kammern des Systems sind so gestaltet, daß sie einen wesentlichen Teil des Teststreifens umgeben und mehrere Kammern untereinander starr verbunden sind. Erfindungsgemäß befinden sich die Kammern daher in einer geometrisch regelmäßigen Anordnung, die bei sachgemäßer Verwendung des Systems nicht deformiert wird, wie dies beispielsweise bei Aluminium-Laminaten der Fall ist. Bevorzugt weist eine einzelne Kammer die Form einer Röhre mit im wesentlichen drei- oder mehreckigem, rundem oder ovalem Querschnitt auf Besonders bevorzugt sind Kammern mit rechteckigem Querschnitt. Der Querschnitt der Kammern besitzt bevorzugt eine Gestalt, die das Testelement entlang seiner Längsachse ummantelt, jedoch nicht an allen Seiten des Testelementes direkt anliegt, so daß eine Bewegung des Testelementes in ein oder zwei Raumrichtungen möglich ist. Die Kammern eines erfindungsgemäßen Vorratsbehältnisses grenzen bevorzugt aneinander, wodurch sowohl Platz als auch Material eingespart werden kann.

Unter einer geometrisch regelmäßigen Anordnung soll im Rahmen der Erfindung eine Anordnung verstanden werden, die dadurch entsteht, daß ein Bauelement existiert, das mehrfach aneinandergereiht das Vorratsbehältnis ergibt.

Ein erfindungsgemäßes Vorratsbehältnis kann aus Materialien gefertigt sein, welche die Eigenschatten Formstabilität und geringe Durchlässigkeit für Wasserdampf verknüpfen. Aus der großen Zahl von Materialien, die diese zwei Eigenschaften miteinander verbinden, sind Materialien bevorzugt, die leicht in eine, getrennte Kammern aufweisende Form, gebracht werden können. Bevorzugt sind Kunststoffe, wie z. B. Polyethylen und Polypropylen.

Die Kammern des Vorratsbehältnisses weisen zwei Öffnungen auf. Besitzen die Testelemente die Form eines Streifen, so befinden sich die Öffnungen der Kammern bevorzugt gegenüber einer schmalen Kante des Testelementes. Die Öffnungen der Kammern bilden mit der gegenüberliegenden Seite des Testelementes einen Winkel, der zwischen 1° und 45° liegt.

Ein erfindungsgemäßes Vortatsbehältnis weist bevorzugt 10 bis 40 Kammern zur Bevorratung einer entsprechenden Zahl von Testelementen auf.

Die Öffnungen der Kammern sind erfindungsgemäß mit einem wasserdampfundurchlässigen Material versiegelt. Im Rahmen der Erfindung soll unter den Begriffen wasserdampfundurchlässig und wenig wasserdampfdurchlässig eine Materialeigenschaft verstanden werden, die relativ gesehen werden muß. Sie kennzeichnet die Brauchbarkeit entsprechender Materialien, ein System zur Verfügung zu stellen, in dem Testelemente über eine Dauer von Monaten aufbewahrt werden können, ohne daß eine inakzeptable Verfälschung des Testergebnisses durch Wasserdampf eintritt.

Bevorzugte Materialien zur Versiegelung der Kammern sind Metallfolien, (z. B. Aluminiumfolie) Aluminium-Laminate und Kunststoffe in geeigneter Dicke (in der Regel > 1 mm). Diese Materialien können auf die Öffnungen der Kammern aufgeklebt, aufgeschmolzen oder aufgepreßt werden. Bevorzugt sind einige der Öffnungen benachbart angeordnet, so daß sie gemeinsam durch ein entsprechend größeres Materialstück versiegelt werden können.

Im Inneren der Kammern des Systems befinden sich erfindungsgemäß Testelemente zur Durchführung von Analysen. Da Testelemente, im besonderen Teststreifen, im Stand der Technik umfassend beschrieben sind, werden sie an dieser Stelle nicht näher charakterisiert. Das erfindungsgemäße System kann jedoch besonders vorteilhalt für solche Testelemente eingesetzt werden, die gegen Verbiegung oder Feuchtigkeit empfindlich sind.

Innerhalb der Kammern des Vorratsbehältnisses kann sich ein Trockenmittel befinden, das Feuchte, die durch den Herstellungsprozeß des Systems eingebracht wurde oder Feuchte, die trotz der Verwendung weitgehend wasserdampfdichter Materialien eindringt, aufnimmt. Als Trockenmittel sind im Stand der Technik bekannte, feste Trockenmittel geeignet, besonders geeignet sind Kieselgele und Molekularsiebe.

An der Außenseite der Kammern sind mehrere Vorrichtungen angebracht, mit denen die Versiegelung an einer Öffnung der Kammer durchstochen werden kann. Diese Vorrichtungen dienen auch dazu, das Testelement aus seiner Kammer herauszuschieben. Das Testelement durchstößt beim Herausschieben die Versiegelung an der zweiten Öffnung der Kammer. Vorrichtungen zum Durchstechen der Versiegelung können z. B. Plastikdorne sein, die an der Außenseite der Kammern angebracht sind. Ein Durchstechen kann erfolgen, indem der jeweilige Dorn so gedreht wird, daß er sich um eine Achse bewegt, die durch seine Verbindungskante mit dem Vortatsbehältnis gegeben ist.

Bei einer weiteren Ausführungsform ist ein Dorn zum Durchstechen der Versiegelung so an dem Vorratsbehaltnis angebracht, daß er von Kammer zu Kammer bewegt wird und zum Durchstechen der jeweiligen Kammer dient, an der er sich befindet.

Auf dem System kann an einer der Außenseiten ein Datenträger angebracht sein, auf dem chargenspezifische Daten der Testelemente und weitere zur Durchführung einer Analyse wichtige Daten, wie z. B. Verfallsdatum und Zahl der Streifen im System, gespeichert sind. Die Daten können auch alphanumerisch in Form eines Strichcodes oder in Form eines Magnetstreifens vorliegen.

Die Erfindung beinhaltet weiterhin ein Verfahren zum zur Verfügung stellen von Testelementen, bei dem eine verschlossene Öffnung einer Kammer durchstochen wird und auf das in der Kammer befindliche Testelement ein mechanischer Druck ausgeübt wird, der das Testelement aus der Kammer hinausschiebt.

Ein erfindungsgemäßes System ermöglicht eine Einzelversiegelung von Testelementen, das bedeutet, daß die Versiegelung eines einzelnen Testelements aufgebrochen werden kann, während die Versiegelung der übrigen im System enthaltenen Testelemente intakt bleibt.

Besonders bevorzugt ist ein Verfahren, bei dem eine erste Öffnung von einem Dorn oder Hebel durchstochen wird und das in der Kammer befindliche Testelement durch eine Weiterbewegung des Dorns oder Hebels auf eine zweite Öffnung hinbewegt wird. Wird durch den Dorn oder Hebel ein ausreichender Druck auf das Testelement ausgeübt, so durchsticht dieses die zweite versiegelte Öffnung und tritt aus dem Vorratsbehältnis aus. Ein Durchstechen der zweiten Versiegelung durch das Testelement kann besonders leicht erfolgen, wenn die Versiegelung und die ihr gegenüberliegende Kante des Testelementes einen spitzen Winkel miteinander bilden. In dieser Anordnung wird die Versiegelung zunächst durch eine Ecke des Testelementes angestochen und von der nachfolgenden Kante weiter aufgeschlitzt.

Ein erfindungsgemäßes System bietet gegenüber bestehenden Verpackungen den Vorteil, daß Testelemente einzeln sowohl manuell als auch durch eine Vorrichtung entnommen werden können, so daß die im System verbleibenden Testelemente weiterhin vor dem Zutritt von Feuchte geschützt sind. Die Entnahme von Teststreifen aus erfindungsgemäßem System kann wesentlich einfacher erfolgen als bei bereits existierenden Verpackungen.

Die Erfindung wird durch zwei Ausführungsformen in den Figuren 1 bis 3 illustriert. Figur 4 zeigt die Funktionsweise einer Vorrichtung zur Entnahme von Testelementen aus einem System nach Ausführungsbeispiel I.

### Ausführungsbeispiel I

- Figur 1:: System in perspektivischer Darstellung
- Figur 2:: Vorratsbehältnis mit Testelementen in seitlicher Ansicht

### Ausführungsbeispiel II

- Figur 3:: System im Querschnitt

### Ausführungsbeispiel I

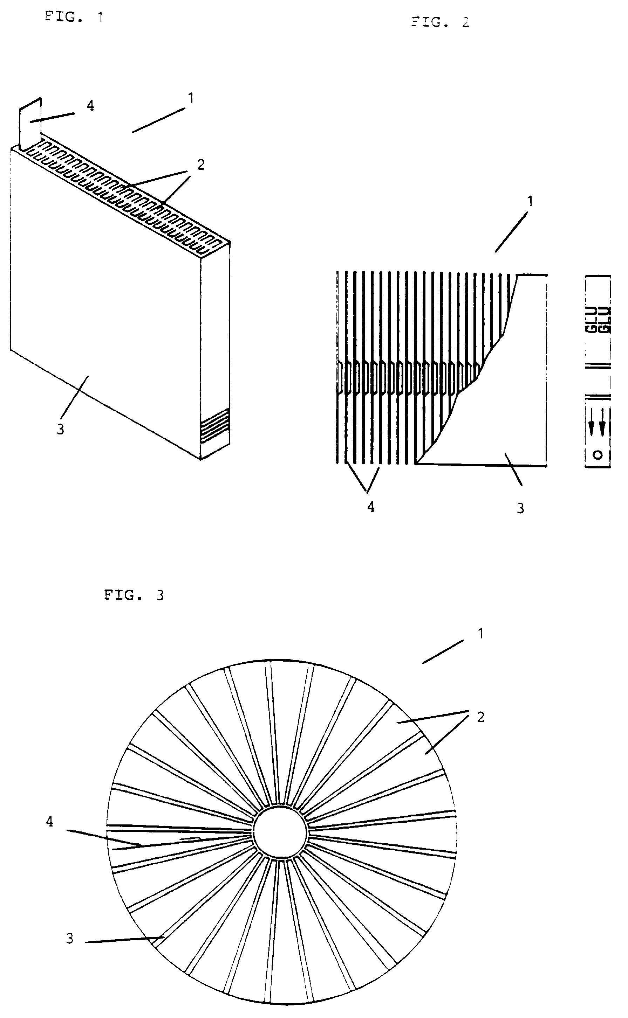

Die Figur 1 zeigt ein erfindungsgemäßes System (1) in perspektivischer Ansicht. Die Kammern (2) des Vorratsbehältnisses (3) besitzen die Form von Röhren mit rechteckigem Querschnitt. In den Kammern (2) befindliche Testelemente (4) sind parallel hintereinander angeordnet. Das Vorratsbehältnis besteht aus Polyethylen und wurde im Spritzgußverfahren gefertigt. Eine Folie aus Aluminium-Laminat zur Versiegelung der Öffnungen der Kammern an Ober- und Unterseite des Vorratsbehältnisses (3) wurde mit einer Ultraschall-Schweißtechnik aufgebracht.

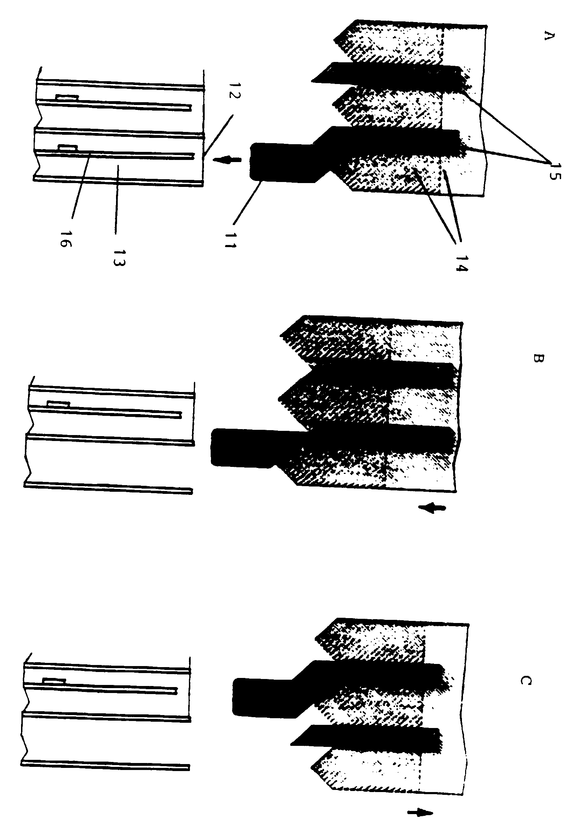

Figur 2 zeigt das erfindungsgemäße System (1) in seitlicher Ansicht bei aufgeschnittenem Vorratsbehältnis (3). In dieser Darstellung ist besonders die platzsparende Verpackung der Testelemente (4) durch eine Anordnung hintereinander zu erkennen.

### Ausführungsbeispiel II

In Figur 3 ist ein System (1) dargestellt, bei dem die Querschnitte der Kammern (2) die Form von Kreissegmenten besitzen. Die länglichen Testelemente (4) können entweder so wie in der Zeichnung angeordnet sein, daß die Längsachse der Testelemente (4) vom Kreiszentrum nach außen weist, oder so, daß die Kreisachse des Vorratsbehältnisses (3) und Längsachse der Testelemente (4) parallel angeordnet sind. Im ersten Fall besitzt das System die Form einer relativ flachen Kreisscheibe und im zweiten Fall die Form eines länglichen Zylinders.

Vorrichtung zur Entnahme von Testelementen:

Figur 4 zeigt schematisch die Funktionsweise einer Vorrichtung zur Entnahme von Testelementen aus einem System (1). Ein Stößel (11) ist gegenüber der versiegelten Öffnung (12) einer Kammer (13) angeordnet. Die Betätigung der Vorrichtung erfolgt, indem aufden Mantel (14) der Entnahmevorrichtung Druck in Richtung auf die Öffnung (12) ausgeübt wird (Figur 4A). Der Stößel (11) durchsticht die Versiegelung der Öffnung (12) und schiebt ein Testelement (16) aus der Kammer (13) heraus. Während sich der Mantel (14) bewegt, bleiben die Führungsstifte (15) an ihrem Ort, was dazu führt, daß Mantel (14) und Führungsstifte (15) in der Endposition der Bewegung eine Rampe bilden, auf der sich der Stößel (11) seitwärts bewegt (Figur 4B).

Wird der Mantel (14) in seine Ausgangsposition zurückbewegt, so bildet sich eine zweite Rampe, auf der sich der Stößel (11) nochmals in die gleiche Richtung seitwärts bewegt und in eine neue Ruheposition einrastet (Figur 4C). Die Abstände von Zapfen des Mantels (14) und Führungsstiften (15) sind so bemessen, daß sich der Stößel nach den Bewegungsabläufen A, B, C gegenüber einer neuen Kammeröffnung befindet.

### Bezugszeichenliste

- (1):: System
- (2):: Kammer
- (3):: Vorratsbehältnis
- (4):: Testelement
- (11):: Stößel
- (12): Versiegelung
- (13):: Kammer
- (14):: Mantel
- (15):: Führungsstift
- (16): Testelement

## Patentansprüche

1. System (1) zur Bevorratung und zum Zurverfügungstellen von Testelementen (4) zur Analyse von Probeflüssigkeiten, bei dem sich zwei oder mehr Testelemente einzeln in wasserdampfdichten Kammern (2) eines Vorratsgefäßes (3) aus einem starren Material befinden und die Kammern (2) die Form von Röhren aufweisen, deren Längsachsen parallel angeordnet sind und die am einen Ende eine erste und am anderen Ende eine zweite Öffnung (12) aufweisen, die durch eine erste und eine zweite Folie verschlossen sind, so daß der Innenraum der Kammern gegen Wasserdampfaus der Umgebung versiegelt ist sowie ein oder mehrere Vorrichtungen (11, 14) an der Außenseite der Kammern, mit denen die erste Folie durchstochen wird und mit der das in der jeweiligen Kammer bevorratete Testelement aus der Kammer herausgeschoben wird, wobei es die zweite Folie durchstößt und die der zweiten Folie gegenüberliegende Seite des Testelementes mit der zweiten Folie einen Winkel zwischen 1° und 45° bildet.

2. System (1) nach Anspruch 1, bei dem die Öffnungen der Kammern (2) durch Folie aus Aluminium oder Aluminiumlaminat, das aufgeklebt, aufgepresst oder aufgeschweißt ist, wasserdampfdicht verschlossen sind.

3. System gemaß Anspruch 1, bei dem die Kante eines Testelementes und die dieser Kante gegenüberliegende verschlossene Öffnung einen Winkel bilden, der größer ist als 5° und kleiner ist als 45°.

4. System gemäß Anspruch 1, bei dem in den Kammern des Systems ein Trockenmittel vorhanden ist.

5. System nach Anspruch 1, bei dem aufdem Vorratsbehältnis ein Datenträger angebracht ist.

6. System gemäß Anspruch 1, bei dem die Vorrichtung zum Durchstechen der Versiegelungsfolie ein Dorn ist, der von Kammer zu Kammer bewegt wird und zum Durchstechen der ersten Folie derjeweiligen Kammer dient, an der er sich befindet.

7. System (1) nach Anspruch 1, bei dem die Vorrichtung zum Durchstechen einen Stößel (11), Führungsstifte (15) und einen Mantel (14) beinhaltet, wobei mit dem Mantel über die Führungsstifte Druck auf den Stößel ausgeübt wird, so daß der Stößel die Versiegelungsfolie durchstößt.

8. System (1) gemäß Anspruch 1, bei dem die Querschnitte der Kammern die Form eines Kreissegmentes besitzen und die Kammern so angeordnet sind, daß das Vorratsbehältnis die Form eines Zylinders besitzt.

9. Verfahren zum Zurverfügungstellen von Testelementen,
die sich einzeln in röhrenförmigen Kammern (2) eines starten Vorratsgefäßes (3) befinden, die Langsachsen der Kammern parallel angeordnet sind und an beiden Enden Öffnungen (12) aufweisen, die durch eine Folie verschlossen sind,
bei dem eine erste Folie, mit der die erste Öffnung einer Kammer verschlossen ist, durchstochen wird und auf das in der Kammer befindliche Testelement ein mechanischer Druck ausgeübt wird, der das Testelement aus der Kammer hinausschiebt und das Testelement bei seinem Austritt aus der Kammer eine zweite Folie, die die zweite Öffnung der Kammer verschließt, durchsticht.

10. Verfahren gemäß Anspruch 9, bei dem die zweite Folie und die ihr gegenüberliegende Kante des Testelementes einen spitzen Winkel bilden.

11. Verfahren gemäß Anspruch 10, bei dem der Winkel zwischen 1° und 45° liegt.

12. Verfahren gemäß Anspruch 9, bei dem das Durchstechen der ersten Folie zum Verschluß der ersten Öffnung mit einem Dorn erfolgt und der Dorn von Kammer zu Kammer bewegt wird, um die jeweiligen Versiegelungsfolien zu durchstechen.

13. Verfahren gemäß Anspruch 9, bei dem Testelemente direkt aus dem Vorratsbehältnis in ein Gerät zur Analyse von Probeflüssigkeiten eingeführt werden.

## Claims

1. System (1) for storing and dispensing test elements (4) for analyzing sample liquids in which two or more test elements are individually located in water-vapour-tight chambers (2) of a storage container (3) made of a rigid material and the chambers (2) have a tubular shape with their longitudinal axes arranged parallel to one another and at one end they have a first opening and at the other end a second opening (12) which are closed by means of a first and second foil so that the inside space of the chambers is sealed against water vapour from the environment and there are one or several devices (11, 14) on the outside of the chambers to pierce the first foil and push the test element stored in the respective chamber out of the chamber in the process of which the element pierces the second foil and the side of the test element opposite to the second foil forms an angle between 1° and 45° to the second foil.

2. System (1) as claimed in claim 1 in which the openings of the chambers (2) are sealed water-vapour-tight by means of a foil made of aluminium or aluminium laminate which is glued, pressed or welded on.

3. System as claimed in claim 1 in which the edge of a test element and the sealed opening opposite this edge form an angle which is greater than 5° but smaller than 45°.

4. System as claimed in claim 1 in which a desiccant is present in the chambers of the system.

5. System as claimed in claim 1 in which a data carrier is affixed to the storage container.

6. System as claimed in claim 1 in which the device for piercing the sealing foil is a spike which is moved from chamber to chamber and is used to pierce the first foil of the respective chamber where it is located.

7. System (1) as claimed in claim 1 in which the piercing device comprises a ram (11), guide pins (15), and a casing (14), wherein pressure is exerted on the ram when the casing is over the guide pins such that the ram pierces the sealing foil.

8. System (1) as claimed in claim 1 in which the cross sections of the chambers have the shape of a circular segment and the chambers are arranged such that the storage container has the shape of a cylinder.

9. Method for dispensing test elements
which are individually located in tubular shaped chambers (2) of a rigid storage container (3), the longitudinal axes of the chambers are parallel to one another and have openings (12) at both ends that are sealed by a foil,
wherein a foil that seals the first opening of a chamber is pierced and mechanical pressure is exerted on the test element contained in the chamber which pushes the test element out of the chamber and the test element pierces a second foil that seals the second opening of the chamber when exiting the chamber.

10. Method as claimed in claim 9 in which the second foil and the opposite edge of the test element form an acute angle.

11. Method as claimed in claim 10 in which the angle is between 1° and 45°.

12. Method as claimed in claim 9 in which the first foil which seals the first opening is pierced with a spike and the spike is moved from chamber to chamber in order to pierce the individual sealing foils.

13. Method as claimed in claim 9 in which test elements are inserted directly from the storage container into an instrument for analyzing sample liquids.

## Revendications

1. Système (1) pour l'approvisionnement et pour la mise à disposition d'éléments d'essais (4) à des fins d'analyse de liquides échantillons, dans lequel deux éléments d'essais ou plus se trouvent à titre individuel dans des chambres étanches à la vapeur d'eau (2) d'un récipient de réserve (3) constitué d'une matière rigide, les chambres (2) présentant la forme de tubes dont les axes longitudinaux sont disposés en parallèle et qui présentent, à une première extrémité, une première et, à l'autre extrémité, une seconde ouverture (12) qui sont fermées par une première et une seconde feuille, si bien que l'espace interne des chambres est rendu étanche à la vapeur d'eau provenant de l'environnement, et dans lequel un ou plusieurs dispositifs (11, 14) sont montés sur le côté externe des chambres avec lesquels la première feuille est transpercée et avec lesquels l'élément d'essai approvisionné dans la chambre respective est poussé hors de la chambre; ce faisant, il transperce la seconde feuille et le côté de l'élément d'essai opposé à la seconde feuille forme avec la seconde feuille un angle entre 1° et 45°.

2. Système (1) selon la revendication 1, dans lequel les ouvertures des chambres (2) sont fermées de manière étanche à la vapeur d'eau à l'aide d'une feuille en aluminium ou en un stratifié d'aluminium, qui est appliquée par collage, par pression ou par soudage.

3. Système selon la revendication 1, dans lequel l'arête d'un élément d'essai et l'ouverture fermée opposée à cette arête forment un angle qui est supérieur à 5° et qui est inférieur à 45°.

4. Système selon la revendication 1, dans lequel un dessicateur est présent dans les chambres du système.

5. Système selon la revendication 1, dans lequel un support de données est appliqué sur le récipient de réserve.

6. Système selon la revendication 1, dans lequel le dispositif pour transpercer la feuille d'étanchéification est une épine qui se déplace de chambre à chambre et qui sert à transpercer la première feuille de la chambre respective, contre laquelle elle se trouve.

7. Système (1) selon la revendication 1, dans lequel le dispositif que l'on utilise pour transpercer renferme un coulisseau (11), des broches de guidage (15) et une gaine (14), les broches de guidage exerçant une pression sur le coulisseau avec la gaine, si bien que le coulisseau transperce la feuille d'étanchéification.

8. Système (1) selon la revendication 1, dans lequel les sections transversales des chambres sont en forme de segment de cercle et les chambres sont disposées de telle sorte que le récipient de réserve est de forme cylindrique.

9. Procédé pour la mise à disposition d'éléments d'essais
qui se trouvent, à titre individuel, dans des chambres (2) de forme tubulaire d'un récipient de réserve rigide (3), les axes longitudinaux des chambres étant disposés en parallèle et les chambres présentant des ouvertures (12) à leurs deux extrémités, qui sont fermées par une feuille,
dans lequel une première feuille, avec laquelle la première ouverture d'une chambre est fermée, est transpercée et exerce sur l'élément d'essai se trouvant dans la chambre, une pression mécanique qui pousse l'élément d'essai hors de la chambre, l'élément d'essai transperçant lors de son évacuation de la chambre une seconde feuille qui ferme la seconde ouverture de la chambre.

10. Procédé selon la revendication 9, dans lequel la seconde feuille et l'arête de l'élément d'essai qui lui est opposée forment un angle aigu.

11. Procédé selon la revendication 10, dans lequel l'angle se situe entre 1° et 45°.

12. Procédé selon la revendication 9, dans lequel la première feuille destinée à la fermeture de la première ouverture est transpercée avec une épine et l'épine se déplace de chambre à chambre pour transpercer les feuilles d'étanchéification respectives.

13. Procédé selon la revendication 9, dans lequel on introduit des éléments d'essais directement à partir du récipient de réserve dans un appareil à des fins d'analyse de liquides échantillons.
